# EUROPEAN PATENT APPLICATION

(11) **EP 4 640 206 A1**
(43) Date of publication of application: **29.10.2025**
(21) Application number: 24172067.1
(22) Date of filing: 24.04.2024
(51) Int. Cl.: A61K 8/64, A61Q 11/00

(54) **NISIN FOR INHIBITING BIOFILMS**

(71) Applicant: SkyLab AG, 1066 Epalinges (CH)
(72) Inventor: Belous, Elena Yur'evna, 121309 MOSCOW (RU); Yarovaya, Alina Olegovna, 109518 MOSCOW (RU)
(74) Representative: Glawe, Delfs, Moll

(57) **Abstract**

The invention relates to a dental and/or oral care product comprising between 0.0039% and 0.0078% by weight of nisin establishing and/or maintaining a pH of at least 4.5 in situ. Although said rather low nisin concentration does not provide an anti-bacterial effect, it is surprisingly and advantageously capable of maintaining stable pH values of at least 4.5, thereby preventing destruction of a subject's teeth enamel based on enamel demineralization effects at pH below 4.5, and said low nisin concentration advantageously further avoids dysbacteriosis, i.e. the oral microflora remains balanced, thus not leading to an increase of e.g. periodontitis associated bacteria.

## Description

### FIELD OF THE INVENTION

### PRIOR ART

Dental caries has been and remains one of the most serious public health problems in recent years; it is the most common disease worldwide (Rathee & Sapra, 2024). At present, health care systems in most countries are focused on provision of dental services to the population; the market of anti-caries oral hygiene products is packed with various items. Despite all the efforts made by governments and manufacturers of household products, about 2.3 billion people worldwide still suffer from caries according to the World Health Organization (WHO). According to the estimates by the WHO, caries affects 50.1% of adult population in the European region (WHO: "Global Oral Health Status Report: Towards Universal Health Coverage for Oral Health by 2030. Summary of the WHO European Region").

Dental caries is one of the major oral diseases; it causes pain and infection. The consequence of severe dental caries is tooth loss, which negatively affects aesthetics, function, self-esteem and quality of life of an individual (Chan et al., 2021). Dental caries is a biofilm-mediated, sugarinduced, multifactorial, dynamic disease that leads to gradual demineralization and remineralization of dental hard tissues. The dynamic process of dental caries consists of rapidly alternating periods of demineralization and remineralization of teeth, which result in specific carious lesions in specific anatomical predispositions of teeth, if complete demineralization occurs for sufficient time(Pitts et al., 2017).

Dental caries usually starts on the enamel surface and below it (initial demineralization occurs below the enamel surface); it is the result of the process, in which the crystalline mineral structure of the tooth is demineralized by organic acids produced by biofilm bacteria as a result of the metabolism of food fermentable carbohydrates, primarily sugars. Despite the fact that microorganisms, which form biofilm in teeth, are able to produce a wide range of organic acids, lactic acid is the predominant end product of the metabolism of sugars and is considered the primary acid involved in caries formation. As acids are accumulated in the liquid phase of the biofilm, pH drops to the point where conditions at the biofilm-enamel border become unsaturated and acid partially demineralizes the surface layer of the tooth. The loss of mineral leads to an increase in porosity, widening of gaps between enamel crystals and softening of the surface, allowing acids to penetrate deeper into the tooth; it results in demineralization of the mineral below the surface (subsurface demineralization (Warreth, 2023).

A decrease in pH in the area of carious lesion leads to changes in microbiota composition. The ability of bacteria to produce alkalis, decompose and produce acids is directly related to the pH of the environment. It is found that at pH 4.5, bacterial diversity of the oral cavity decreases significantly, acid-producing bacteria begin to predominate, and non-pathogenic bacteria are killed, which increases the destructive effect on dentin and accelerates the development of cariogenic processes (McLean et al., 2012). Thus, the process of acid production and acidification of the environment is the cornerstone in the pathogenesis of caries development.

It has been generally accepted for a long time that Streptococcus mutans and Streptococcus sobrinus are dental caries agents; these are bacteria, the metabolic products of which are acids. These observations led to the assumption that caries was caused by only a limited subset of the many species found in dental biofilms. However, as additional epidemiologic studies were conducted, caries was also observed in the distinct absence of these bacteria, while these organisms could persist on other surfaces that remained healthy, assuming that other bacterial species also play an important role in caries initiation and biofilm community interactions (Simón-Soro et al., 2014). In one of the studies, when comparing two groups of patients: a control group (subjects, not suffering from caries), and an experimental group (subjects with extensive carious lesions), predominance of S.mutans in the affected areas was observed. However, in about 15% subjects of the test group, microflora in the lesions either did not contain streptococci at all or the amount was within the normal range (Sansone et al., 1993). It allows to assume that other bacteria may also be involved in the pathogenesis of dental caries, which have not been focused on before.

Most recently, studies using traditional culture-based or molecular approaches have detected associations between dental caries and other groups of acid-producing and acid-resistant bacteria, including a number of Bifidobacterium spp., Actinomyces spp., Propionibacterium spp. and Scardovia wiggsiae (Pitts et al., 2017). These and other microorganisms associated with oral diseases are endogenous species rather than pathogens introduced from the environment. Consequently, strategies for caries treatment and prevention should be aimed at controlling the growth of these organisms and maintaining normal oral conditions. Antibacterial agents designed to completely remove potentially harmful species may lead to oral dysbiosis (Rebelo et al., 2023).

At present, the basic main methods of caries management are the use of various remineralizing and antibacterial agents in oral care products, which inhibit the development of biofilms and suppress the growth of pathogenic bacteria such as S. mutans or S. sobrinus. Antibacterial agents are usually substances such as chlorhexidine, triclosan, bacterial peroxidases, extracts and oils of medicinal plants. Although these substances have repeatedly shown their antimicrobial activity in studies, they have a significant disadvantage that challenge their use in oral products.

Studies have shown that that prolonged use of chlorhexidine as an antimicrobial agent leads to oral dysbacteriosis. Lack of specificity only to pathogenic bacterial species results in imbalance of oral microflora. For example, the use of chlorhexidine significantly reduces not only the population of pathogenic *C.albicans* but also the population of *Firmicutes* (Neves et al., 2021). Moreover, it has been shown that the efficacy of chlorhexidine treatment significantly depends on the initial microbiota composition and in some cases can lead to worsening of the disease, due to disruption of symbiotic microbiome (Chatzigiannidou et al., 2020). In vivo study showed that chlorhexidine treatment reduced the microbial load than as compared to non-treatment, but this was done by means of non-selective effect on the oral microbiome, leading to an increase in the relative abundance of several taxa associated with periodontitis (e.g. *Fusobacterium*) (Al-Kamel et al., 2019).

Triclosan, another broad-spectrum antimicrobial agent, is widely used in personal care products, medical devices, plastic cutting boards and food storage containers. Given its strong lipophilicity and ability to accumulate in organisms, it is potentially harmful to biological health. It is also classified as one of the chemical substances that can disrupt the endocrine system. In September 2016, the USA Food and Drug Administration (FDA) and the European Union prohibited the use of triclosan in soap products. Triclosan is able to inhibit the growth of a large number of gram-positive and gram-negative bacteria; however, the use of triclosan has been shown to lead to the development of resistance in bacteria and the decrease of sensitivity to other antimicrobial agents (Rozman et al., 2021). Long-term use of toothpastes containing triclosan (over 2 weeks) resulted in significant changes in the oral microflora and a decrease in the number of viable bacteria (Urazova R. et al, 2009).

In recent years, products containing the enzymes, peroxidases, have appeared on the market. Numerous *in vitro* studies have demonstrated antibacterial, antifungal and antiviral role of peroxidases, making them potentially attractive antimicrobial agents (Courtois, 2021). However, the introduction of inactive peroxidase into the oral cavity has been shown to disrupt the in vitro balance between commensals and periodontopathogens in favor of the latter (Herrero et al., 2018).

A trend towards the use of eco-friendly products with natural and safe composition resulted in the availability of a large number of compositions that include plant components, oils or extracts. Polyphenols, alkaloids and flavonoids are able to perform significant antibacterial activity against caries pathogens. However, regular intake of green tea polyphenols, for example, has been shown to irreversibly alter the oral microbiota, including increased levels of Bifidobacterium, which under certain conditions are also able to form biofilms that lead to the development of caries (Yuan et al., 2018). Moreover, it has been shown that despite the ability of green tea extract to reduce the number of S. mutans, at the same time it promotes the growth of Porphymonas gingivalis population and does not affect the levels of bacteria of the Fusobacterium genus, which are participants in periodontal disease (Adami et al., 2018). This suggests that the reduction of one bacterial species may lead to unpredictable consequences such as stimulation of the growth of other pathogenic species, etc.

For this reason, there is a distinct necessity of alternative approaches to the selection of anti-caries agents that do not indiscriminately target the oral microbiome, but specifically and selectively target pathogenic strains, or use other mechanisms to prevent the development of cariogenic processes.

Nisim, an active component, is used in the present invention, which aims at controlling dental caries without disturbing the balance of the oral microflora by maintaining a normal buffer balance in the oral microbial biofilm.

Nisin is an antimicrobial peptide of bacterial origin and is a tasteless and odorless substance. It is a cationic, amphiphilic, antimicrobial polypeptide synthesized by ribosomes and modified post-translationally to its biologically active form. It is included into bacteriocins classified as type A lantibiotics (Aveyard et al., 2017; Bin Hafeez et al., 2021). Nisin is mainly produced by gram-positive bacteria, which include *Lactococcus* and *Streptococcus* species (e.g., *Lactococcus lactis* (*L. lactis*)*, Streptococcus hyointestinalis.* Nisin is primarily known for its broad-spectrum antibacterial activity against a wide range of gram-positive *Lactococcus, Enterococcus,* Streptococcus, *Staphylococcus, Listeria* and *Micrococcus* bacteria, as well as vegetative forms and proliferating spores of *Bacillus* and *Clostridium.* Nisin is even better than conventional antibiotics due to its stability at higher temperature, tolerance to low pH and dual mode antimicrobial activity (Pandey et al., 2020; Shin et al., 2015). The latter involves binding of a nisin molecule to cell wall lipid, which leads to the formation of a pore-forming complex within the cell membrane, thus causing leakage of important cellular components and ultimately cell death (Bahrami et al., 2019).

In light of its biomedical potential, nisin has already shown promising results as an alternative to conventional antimicrobial therapy due to its activity against specific (antibioticresistant bacteria) pathogens and pathological conditions. For example, the inhibitory ability of nisin against S. aureus, the main causative agent of atopic dermatitis, has been demonstrated.

In addition, nisin has previously shown its potential in oral diseases such as dental caries and periodontal disease through inhibition of oral bacteria including *Streptococcus sanguinis, Streptococcus sobrinus, Streptococcus gordonii, P. gingivalis, P. intermedia, A. actinomycetemcomitans* and *T. Denticola* (Radaic et al., 2020; Shin et al., 2015). The antimicrobial efficacy of nisin against the formation of multispecies oral biofilms derived from saliva was also studied. It was reported to reduce biofilm biomass in a dose-dependent manner (*Shin J. et al., 2015).* In addition, nisin has demonstrated to have potential in the treatment of nonhealing wounds as it increases skin cell motility, attenuates the action of lipopolysaccharides and pro-inflammatory cytokines and reduces the bacterial load in the wound (Jančič & Gorgieva, 2022). Antimicrobial activity, solubility and thermostability of nisin are higher at acidic pH and are deactivated under alkaline conditions due to irreversible structural changes in the nisin molecule. Nisin has higher antimicrobial activity in liquid medium than in solid medium. Nisin is highly stable at low temperatures (e.g., freezing) but loses activity with prolonged heating (Qian et al., 2021).

According to most authors, the advantages of nisin are: inhibitory efficacy against a wide range of microorganisms, low probability of development of resistance of microorganisms, absence of effect on normal intestinal microbiota, nontoxicity, colorlessness and tastelessness.

Thus, numerous studies have previously disclosed the exclusively antibacterial properties of nisin. However, it was unexpectedly discovered by the authors of the present invention that nisin in certain concentrations does not exhibit antibacterial effects, but is capable of maintaining a stable pH level, preventing it from dropping to critically low values dangerous to enamel. Maintenance of the pH level will allow the prevention of destruction of hydroxyapatite with metabolites of various acid-forming bacteria such as S. mutans, and, accordingly, will stop the development of caries.

The prior art discloses Oralpeace "Outdoor adventure" toothpaste made of natural food ingredients. The composition of this toothpaste comprises nisin, included into the formula of the patented component Neonisin-e^{®} (Patent No. 5750552); in addition to nisin, the composition includes plum extract and Damask rose essential oil. Manufacturers offer to use this product to remove plaque, prevent tooth decay and destroy bad breath. This toothpaste is primarily aimed at combating a wide range of microorganisms due to the added herbal components. It can be assumed that this component is not specific to pathogenic bacteria and may lead to an imbalance of the microbiota in the oral cavity.

The prior art discloses a composition containing nisin, which is designed for oral care (see patent US20200337975A1b, published on 2020-10-29). Sodium bicarbonate and nisin are claimed as the main components; when administered in the oral cavity, they promote breakdown and removal of biofilms. However, during experiments performed by the authors of the composition it was shown that the nisin solution without the addition of sodium bicarbonate is not able to inhibit the growth of biofilms. It can be assumed that the main active ingredient of the composition is sodium bicarbonate, which manifests the indicated properties of this product. In addition, the authors do not consider the possibility of using the composition as an anti-caries agent. Thus, it is likely that the addition of nisin to the composition has mainly a marketing function.

The prior art also discloses a composition comprising nisin solution, humidifier, metal ion chelator and flavoring agent (see patent WO1996037181A1, published on 1996-05-22). This invention includes nisin as an antibacterial component and is designed to reduce the activity of oral pathogens, including Candida fungi, and controlling candidiasis. It is assumed that this composition may also be useful for healing wounds in the mouth. A disadvantage of this composition is that the authors claim the intended pH of the composition to be in the range of 4 -5.5; however, at pH 4.5, hydroxyapatites of enamel prisms begin to deteriorate, which can lead to enamel depletion and give an advantage to acid-producing bacteria, thereby promoting the initiation of cariogenic processes.

The above-mentioned disadvantages are eliminated in the present invention by the use of low concentrations of nisin, which are unable to have antibacterial effect but are able to maintain a constant pH level. Since the mechanism of action of nisin is not species-specific and is not based on interaction with bacteria, there is every reason to believe that it will inhibit enamel demineralization even regardless of the cause and species of acid metabolite producer.

### DISCLOSURE OF THE INVENTION

### BRIEF DESCRIPTION OF DRAWINGS

### EMBODIMENT

The authors unexpectedly found that the use of nisin in low concentrations is able to ensure the maintenance of pH constancy in the oral cavity. This effect allows preventing the destruction of hydroxyapatite crystals by acids produced by oral pathogens, while, due to the absence of bactericidal action, the component will not cause dysbacteriosis.

Additional beneficial effects and actions understood by persons skilled in the art are disclosed throughout the document or are directly derived from the description of the invention, in particular the experimental data obtained.

### EXAMPLES

Example No. 1. Determination of the effect of different concentrations of nisin on growth and biomass accumulation of *S.mutans No. 22081* in 16-hour follow-up.

Aqueous solution of nisin in different concentrations was used as the working solution.

To prepare the reaction mixture containing the tested component, a method of serial twofold dilutions in heart-brain broth (Series 201181, valid till 012025., CONDALAB, Spain), a micromethod (flat-bottom 96-well plate), for which serial dilutions of the investigated component were prepared in liquid nutrient medium. When setting the methods of serial dilutions, control of culture growth on medium without the active component was carried out, and the quality of medium and investigated preparations was controlled using reference strains of the species. The purity of the microorganism suspension used for inoculation was also controlled by sowing on non-selective media.

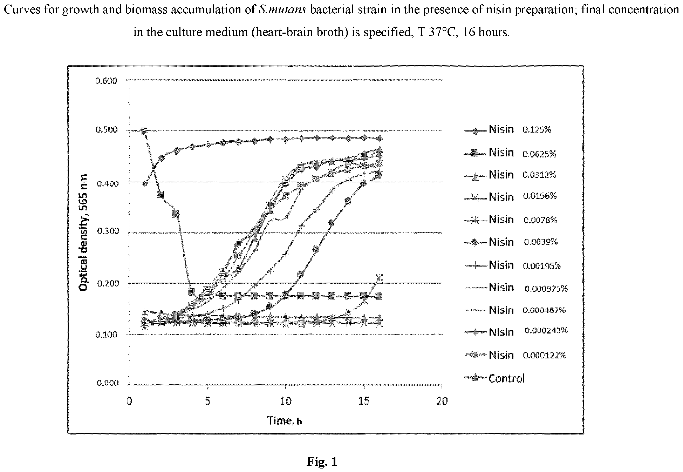

The results of the micromethod formulation to evaluate the effect of drugs on the growth and development of the culture of *Streptococcus mutans No. 22081* were recorded spectrophotometrically in an automatic kinetic format (intermittent shaking, T-37°C, measurement of optical density A565 nm every hour in automatic mode). The results of the suppression of the growth for *S.mutans* bacterial strain are demonstrated in Figure 1.

The results of this test allow us to conclude that nisin in concentrations of 0.0625-0.125% has its own optical density; by the fourth hour of observation in concentration of 0.0625%, optical density decreased, probably due to possible biodegradation of the drug, and subsequently the density did not further change. It can be affirmed that nisin in the final concentration of 0.0078-0.125% has a pronounced antibacterial effect against *S.mutans* strain. In concentrations of 0.0039-0.0078%%, nisin significantly inhibited growth and biomass accumulation, but had no bactericidal effect. Lower concentrations had no significant effect. The concentration of 0.0039% can be considered as the minimum-suppressive concentration.

Example No. 2. Follow-up observation of pH of washes of a laboratory model of *S. mutans* biofilms.

Inoculum of *S.mutans* control strain was prepared from an ampoule. The ampoule with the dried strain was opened sterilely over a drier or a tray. The upper end of the ampoule was heated on a burner flame, and paraffin was removed; then a piece of sterile absorbent cotton moistened in sterile water was carefully touched to the pulled end of the ampoule so that a small crack appeared, and the same wet cotton was passed around the tip of the ampoule. After the crack had formed, the end of the ampoule was removed with a light blow of tweezers. After opening the ampoule, 0.3-0.5 ml of heart-brain broth was sterilely poured in. After dissolution of the dry contents, the ampoule was transferred with a Pasteur pipette into tubes with 2 ml of heart-brain broth and grown at 37°C for 2 hours in a thermostat in 5% CO2 medium. To restore full viability, 1-2 passages on nutrient media appropriate for the microbes of the species were required. 2 Petri dishes with agar containing 5% horse blood were seeded with 0.1 ml of a 2-hour culture on heart-brain broth of the strain. Petri dishes were incubated at 37°C for 18 hours in an incubator containing 5% CO2. Gram stained smears were prepared from the grown colonies to assess the tinctorial, phenotypic properties and homogeneity of bacteria. Species membership was confirmed using MALDI-TOF reference bases.

Aqueous solutions of nisin in concentrations of 0.004 and 0.008% were taken as the working solutions for testing. These concentrations were chosen because, according to the results of the study described in Example 1, they do not have a bactericidal effect and do not significantly alter bacterial growth during the measured time period (30 minutes), as shown in Figure 1. Distilled water was used as a negative control. Moreover, urea solution at a concentration of 0.05% was tested for comparison. Urea was chosen due to its ability to alkalinize the environment in bacterial biofilms (Jadon et al., 2018). During the assay, a 10% sucrose solution was used, which is known to be readily metabolized by cariogenic bacteria to acid, thus contributing to the overall acidification of the medium.

The contents of the wells were extracted using an aspirator. All 6 wells of the first row were filled with 1 ml of deionized water and left for 5 min. The water was extracted with an aspirator; 1 ml of the test solution was added into the wells; in 1 min the component was removed, the wells were glazed for 1 min with deionized water, then it was removed. 250 µl of 10% sucrose was added to the wells and biofilms were scraped off the well walls with a disposable plastic loop. The resulting suspension was transferred by pipette into three Eppendorf tubes: contents of wells 1 and 2 into Eppendorf tube No. 1, contents of wells 3 and 4 into Eppendorf tube No. 2, contents of wells 5 and 6 into Eppendorf tube No. 3; next, the measurement of pH started immediately. Time points of the measurement are: 0, 1, 2, 3, 4, 5, 6, 7, 8, 9, 10, 15, 20, 30 min. After the test was finished, the electrode was washed and the described procedure was repeated for the next solution under study.

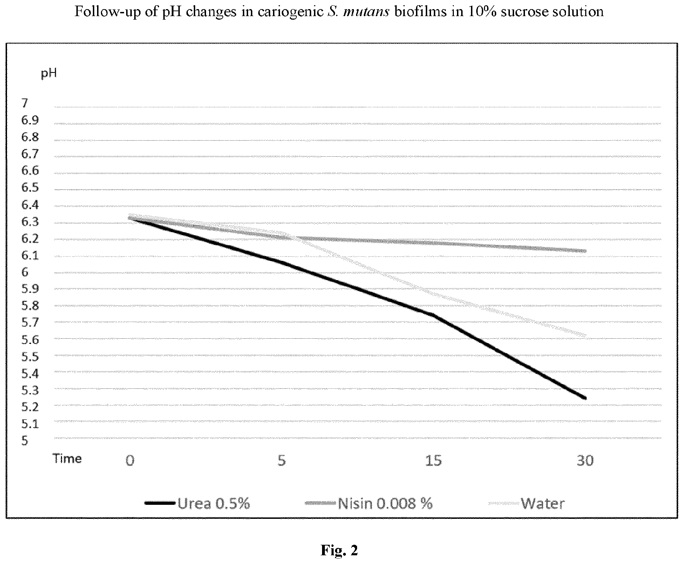

The results of pH changes in cariogenic *S.mutans* biofilms in the presence of sucrose under the influence of active ingredients are demonstrated in Figure 2 and Tables 1-2.

**Table 1. Results of pH measurement of mechanically disrupted S.mutans biofilms in 10% sucrose solution, follow-up (10% sucrose solution pH-6.98).**

| Component name | | | | | | | |
|---|---|---|---|---|---|---|---|
| Time of change, min. | 0 | 1 | 2 | 3 | 4 | 5 | 6 |
| Control | 6.35±0.006 | 6.33±0.006 (-0.02) | 6.29±0.006 (-0.04) | 6.28±0.01 (-0.01) | 6.26±0.006 (-0.02) | 6.24±0 (-0.02) | 6.21±0.03 (-0.03) |
| Urea 0.5% | 6.33±0.03 | 6.32±0.006 (-0.01) | 6.22±0.01 (-0.1) | 6.15±0.006 (-0.07) | 6.09±0.006 (-0.06) | 6.06±0 (-0.03) | 6.04±0 (-0.02) |
| Nisin 0.004% | 6.09±0.006 | 6.08±0.006 (-0.01) | 6.07±0.006 (-0.01) | 6.05±0.006 (-0.02) | 6.05±0 (0) | 6.04±0.006 (-0.01) | 6.05±0.006 (+0.01) |
| Nisin 0,008% | 6.33±0 | 6.32±0.006 (-0.01) | 6.31±0.006 (-0.01) | 6.28±0.01 (-0.03) | 6.24±0.006 (-0.04) | 6.21±0.006 (-0.03) | 6.21±0 (-0.03) |

| Component name | | | | | | | |
|---|---|---|---|---|---|---|---|
| Time of change, min. | 7 | 8 | 9 | 10 | 15 | 20 | 30 |
| Control | 6.18±0.006 (-0.03) | 6.16±0.01 (-0.03) | 6.14±0.006 (-0.02) | 6.12±0.01 (-0.02) | 5.87±0.01 (-0.25) | 5.85±0.006 (-0.02) | 5.62±0.03 (-0.23) |
| Urea 0.5% | 6.02±0.006 (-0.02) | 6.0±0.01 (-0.02) | 5.93±0.02 (-0.07) | 5.83±0.02 (-0.1 | 5.74±0.02 (-0.09) | 5.46±0.02 (-0.28) | 5.24±0.006 (-0.22) |
| Nisin 0.004% | 6.05±0.006 (0) | 6.05±0 (0) | 6.05±0 (0) | 6.06±0.006 (+0.01) | 6.05±0.006 (-0.01) | 6.04±0.006 (-0.01) | 6.0±0 (-0.04) |
| Nisin 0,008% | 6.23±0 (+0.02) | 6.23±0 (0) | 6.21±0 (-0.02) | 6.2±0.006 (-0.01) | 6.18±0 (-0.02) | 6.17±0.006 (-0.01 | 6.13±0.006 (-0.04) |

### Figure 2. Values of pH change in cariogenic S. mutans biofilms of in 10% sucrose solution compared to the initial value

| Substance/concentration | Value of pH decrease within 30 min. of measurement (%) |
|---|---|
| Urea 0.5% | -17.22% |
| Nisin 0.004% | -1.47% |
| Nisin 0.008% | -3.15% |
| Water | -11.5% |

As can be seen from the results in Figure 2 and Tables 1-2, nisin has a stabilizing effect on the pH of cariogenic biofilm affected by sucrose. The effect of nisin on pH of the mixture has an inverse dose-dependent effect, i.e. lower concentrations (0.004%) have a more stabilizing effect on the pH of the biofilm as compared to water. However, higher concentrations of nisin (0.008%) have the ability to maintain normal pH of biofilms more effectively than water and urea (0.5%) and prevent it from dropping to the critical pH value of 5.5, when hydroxyapatite crystals are damaged. Nisin in concentrations of 0.004-0.008%, which has no effect on the development or inhibition of growth of *Streptococcus mutans* cariogenic bacteria within the limits of measurements, helps to maintain the pH almost equal to the initial value before the addition of sucrose, a readily fermentable carbohydrate that some bacterial species, including Streptococcus mutans, are able to process to acids that locally lower pH in biofilms. Urea in concentration of 0.5% has no ability to stabilize pH in monospecies cariogenic biofilms despite its alkaline nature as compared to the negative control.

Example No. 3. Follow-up observation of pH of washes of a laboratory model of multispecies biofilms.

To perform this test, it was necessary to obtain multispecies biofilms of bacteria. A 72-well, round-bottom, polystyrene plate for titration was used as an adhesive surface for growing multispecies biofilms. The inner surface was processed with a round abrasive drill bit, with visual inspection of the procedure. Then the plate was washed and immersed into isopropyl alcohol for 10 min. followed by 3-fold washing in distilled water.

Saliva was collected in the fasting state by spitting into a sterile centrifuge tube. Saliva was used to prepare inoculum with the following composition: 96.5 ml of saliva, 1 ml of 10% sucrose, 3.5 ml of S.mutans suspension 0.8 units of McF.

To obtain biofilms, the prepared inoculum was poured 1 ml into all wells of the prepared plate, after which the wells were covered with a similar plate and sealed with parafilm film. The plate was placed on a shaker pad with variable surface slope placed in a CO₂ incubator and incubated overnight (16 hours). The contents of the wells were then extracted with an aspirator by dipping the plastic tip into the center of the well until completely drained. 1 ml of soy-casein broth was added to all wells of the plate with an automatic pipette. The plate was placed into the incubator. The described cycle was repeated for four days.

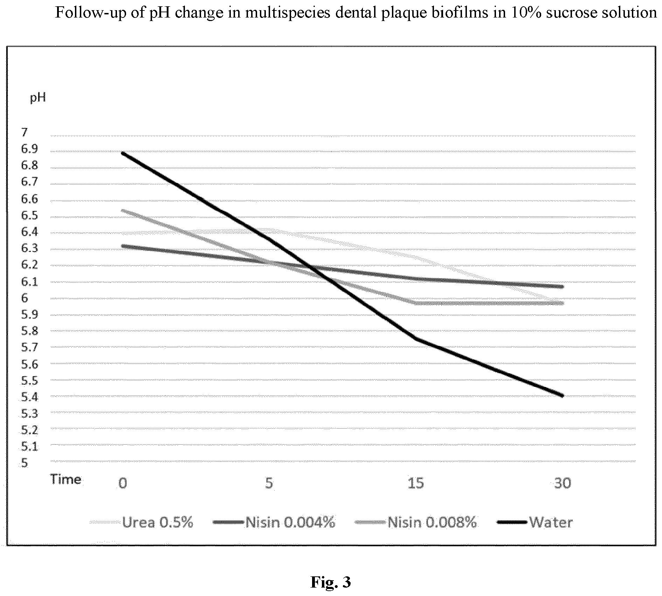

Biofilm formation was monitored visually by gram staining. After four days, the contents of the wells were extracted with an aspirator. All 6 wells of the first row were filled with 1 ml of deionized water and left for 5 min. Water was extracted with an aspirator; 1 ml of the test drug was added into the wells, in 1 min. the drug was removed, the wells were filled for 1 min. with deionized water, then it was removed. 250 µl of 10% sucrose solution was added to the wells. The formed biofilms were scraped off with a disposable loop (10µl); the suspension of two neighboring wells was combined and transferred to three Eppendorfs. Sample combination allowed increasing the volume of the analyzed suspension sample up to 0.5 ml, which in turn allowed carrying out pH measurements in free volume with the necessary reserve at high density of biofilm culture of microorganisms and optimal time for the sample measurement procedure (20 sec.). After combination of the samples, pH was immediately measured. The measurement time points were 0, 1, 2, 3, 4, 5, 6, 6, 7, 8, 9, 10, 15,20,30 min. The results of pH changes in multispecies dental biofilms in the presence of sucrose under the influence of active ingredients are shown in Figure 3 and Tables 3-4.

**Table 3. Results of pH measurements of mechanically disrupted multispecies biofilms in 10% sucrose solution, follow-up (10% sucrose solution pH-6.98).**

| Component name | | | | | | | |
|---|---|---|---|---|---|---|---|
| Time of change, min. | 0 | 1 | 2 | 3 | 4 | 5 | 6 |
| Control | 6.89±0.006 | 6.79±0.006 (-0.1) | 6.67±0.0 (-0.12) | 6.56±0.006 (-0.11) | 6.45±0.006 (-0.11) | 6.36±0.006 (-0.09) | 6.31±0.006 (-0.05) |
| Urea 0.5% | 6.4±0.006 | 6.4±0.006 (0) | 6.4±0.006 (0) | 6.4±0.006 (0) | 6.41±0.006 (+0.01) | 6.42±0.006 (+0.01) | 6.44±0.006 (+0.02) |
| Nisin 0.004% | 6.32±0.006 | 6.3±0.006 (-0.02) | 6.28±0.0 (-0.02) | 6.26±0.006 (-0.02) | 6.24±0.01 (-0.02) | 6.22±0.006 (-0.02) | 6.2±0.0 (-0.02) |
| Nisin 0,008% | 6.54±0.006 | 6.45±0.006 (-0.09) | 6.4±0.006 (-0.05) | 6.35±0.006 (-0.05) | 6.3±0.006 (-0.05) | 6.22±0.01 (-0.08) | 6.16±0.006 (-0.06) |

| Component name | | | | | | | |
|---|---|---|---|---|---|---|---|
| Time of change, min. | 7 | 8 | 9 | 10 | 15 | 20 | 30 |
| Control | 6.24±0.006 (-0.07) | 6.18±0.006 (-0.06) | 6.13±0.0 (-0.05) | 5.89±0.006 (-0.24) | 5.75±0.006 (-0.14) | 5.59±0.01 (-0.16) | 5.4±0.02 (-0.19) |
| Urea 0.5% | 6.45±0.006 (+0.01) | 6.43±0.02 (-0.02) | 6.43±0.0 (0) | 6.42±0.0 (-0.01) | 6.25±0.01 (-0.17) | 6.18±0.02 (-0.07) | 5.97±0.02 (-0.21) |
| Nisin 0.004% | 6.2±0.006 (0) | 6.19±0.0 (-0.01) | 6.19±0.006 (0) | 6.18±0.0 (-0.01) | 6.12±0.006 (-0.06) | 6.11±0.0 (-0.01) | 6.07±0.006 (-0.04) |
| Nisin 0,008% | 6.1±0.01 (-0.06) | 6.0±0.02 (-0.1) | 5.99±0.006 (-0.01) | 5.98±0.0 (-0.01) | 5.97±0.006 (-0.01) | 5.96±0.006 (-0.01) | 5.97±0.006 (+0.01) |

**Table 4. Values of pH change in multispecies dental biofilms in 10% sucrose solution as compared to the initial value**

| Substance/concentration | Value of pH decrease within 30 min. of measurement (%) |
|---|---|
| Urea 0.5% | -6.7% |
| Nisin 0.004% | -3.95% |
| Nisin 0.008% | -8.7% |
| Water | -21.6% |

The results of the test, presented in Figure 3 and Tables 3-4, show that nisin, with the initial pH level comparable to the control, as in the case with monospecies cariogenic biofilm, has a stabilizing effect on the value of acidity in the multispecies biofilm affected by sucrose. Moreover, the effect of nisin on the pH of the mixture, as well as in the case of monospecies biofilm, has an inverse dose-dependent effect that persists throughout the observation period. It can be seen that nisin in concentration of 0.004% has a better effect to maintain normal pH under increased sugar load as compared to the control and also compared to the known active urea, which is able to be converted to ammonia, which has an alkaline pH due to the presence of urease enzyme in some dental plaque bacteria; it is reported in various literature (Kleinberg, 1967). Both active nisin concentrations of 0.004 and 0.008% prevent pH decrease in the plaque biofilm model to a critically low value of 5.5, unlike distilled water.

As can be seen from the results of all the examples, the authors unexpectedly found that nisin solution in concentration of 0.008% and below is not able to have antibacterial and inhibitory activity against S.mutans; however, in these concentrations it is able to stabilize pH, thus preventing oxidation of tooth enamel and neutralizing acids produced by bacteria, which is a fundamentally new mechanism of action of this peptide.

### References

Adami, G. R., Tangney, C. C., Tang, J. L., Zhou, Y., Ghaffari, S., Naqib, A., Sinha, S., Green, S. J., & Schwartz, J. L. (2018). Effects of green tea on miRNA and microbiome of oral epithelium. Scientific Reports, 8(1). https://doi.org/10.1038/s41598-018-22994-3
Al-Kamel, A., Al-Hajj, W. A., Halboub, E., Abdulrab, S., Al-Tahami, K., & Al-Hebshi, N. N. (2019). N-acetyl cysteine versus chlorhexidine mouthwashes in prevention and treatment of experimental gingivitis: a randomized, triple-blind, placebo-controlled clinical trial. Clinical Oral Investigations, 23(10). https://doi.org/10.1007/s00784-019-02813-3
Aveyard, J., Bradley, J. W., McKay, K., McBride, F., Donaghy, D., Raval, R., & D'Sa, R. A. (2017). Linker-free covalent immobilization of nisin using atmospheric pressure plasma induced grafting. Journal of Materials Chemistry B, 5(13). https://doi.org/10.1039/c7tb00113d
Bahrami, A., Delshadi, R., Jafari, S. M., & Williams, L. (2019). Nanoencapsulated nisin: An engineered natural antimicrobial system for the food industry. In Trends in Food Science and Technology (Vol. 94). https://doi.org/10.1016/j.tifs.2019.10.002
Bin Hafeez, A., Jiang, X., Bergen, P. J., & Zhu, Y. (2021). Antimicrobial peptides: An update on classifications and databases. In International Journal of Molecular Sciences (Vol. 22, Issue 21). https://doi.org/10.3390/ijms222111691
Chan, A. K. Y., Tamrakar, M., Jiang, C. M., Lo, E. C. M., Leung, K. C. M., & Chu, C. H. (2021). A Systematic Review on Caries Status of Older Adults. International Journal of Environmental Research and Public Health, 18(20), 10662. https://doi.org/10.3390/ijerph182010662
Chatzigiannidou, I., Teughels, W., Van de Wiele, T., & Boon, N. (2020). Oral biofilms exposure to chlorhexidine results in altered microbial composition and metabolic profile. Npj Biofilms and Microbiomes, 6(1). https://doi.org/10.1038/s41522-020-0124-3
Courtois, P. (2021). Oral peroxidases: From antimicrobial agents to ecological actors (Review). Molecular Medicine Reports, 24(1). https://doi.org/10.3892/mmr.2021.12139
Herrero, E. R., Boon, N., Bernaerts, K., Slomka, V., Verspecht, T., Quirynen, M., & Teughels, W. (2018). Clinical concentrations of peroxidases cause dysbiosis in in vitro oral biofilms. Journal of Periodontal Research, 53(3). https://doi.org/10.1111/jre.12534
Jadon, P., Selladurai, R., Yadav, S. S., Coumar, M. V., Dotaniya, M. L., Singh, A. K., Bhadouriya, J., & Kundu, S. (2018). Volatilization and leaching losses of nitrogen from different coated urea fertilizers. Journal of Soil Science and Plant Nutrition, 18(4). https://doi.org/10.4067/50718-95162018005002903
Jančič, U., & Gorgieva, S. (2022). Bromelain and Nisin: The Natural Antimicrobials with High Potential in Biomedicine. In *Pharmaceutics* (Vol. 14, Issue 1). https://doi.org/10.3390/pharmaceutics14010076
McLean, J. S., Fansler, S. J., Majors, P. D., McAteer, K., Allen, L. Z., Shirtliff, M. E., Lux, R., & Shi, W. (2012). Identifying low pH active and lactate-utilizing taxa within oral microbiome communities from healthy children using stable isotope probing techniques. PLoS ONE, 7(3). https://doi.org/10.1371/journal.pone.0032219
Neves, C. A. das, Alves, C. H., Rocha, N. C., Rizzardi, K. F., Russi, K. L., Palazzi, A. A. A., Parisotto, T. M., & Girardello, R. (2021). Firmicutes Dysbiosis After Chlorhexidine Prophylaxis in Healthy Patients Submitted to Impacted Lower Third Molar Extraction. Frontiers in Cellular and Infection Microbiology, 11. https://doi.org/10.3389/fcimb.2021.702014
Pandey, P., Hansmann, U. H. E., & Wang, F. (2020). Altering the solubility of the antibiotic candidate nisin - A computational study. ACS Omega, 5(38). https://doi.org/10.1021/acsomega.0c03594
Pitts, N. B., Zero, D. T., Marsh, P. D., Ekstrand, K., Weintraub, J. A., Ramos-Gomez, F., Tagami, J., Twetman, S., Tsakos, G., & Ismail, A. (2017). Dental caries. Nature Reviews Disease Primers, 3(1), 17030. https://doi.org/10.1038/nrdp.2017.30
Qian, J., Chen, Y., Wang, Q., Zhao, X., Yang, H., Gong, F., & Guo, H. (2021). Preparation and antimicrobial activity of pectin-chitosan embedding nisin microcapsules. European Polymer Journal, 157. https://doi.org/10.1016/j.eurpolymj.2021.110676
Radaic, A., Ye, C., Parks, B., Gao, L., Kuraji, R., Malone, E., Kamarajan, P., Zhan, L., & Kapila, Y. L. (2020). Modulation of pathogenic oral biofilms towards health with nisin probiotic. Journal of Oral Microbiology, 12(1). https://doi.org/10.1080/20002297.2020.1809302
Rathee, M., & Sapra, A. (2024). Dental Caries*.*
Rebelo, M. B., Oliveira, C. S., & Tavaria, F. K. (2023). Novel Strategies for Preventing Dysbiosis in the Oral Cavity. Frontiers in Bioscience-Elite, 15(4), 23. https://doi.org/10.31083/j.fbel504023
Rozman, U., Pušnik, M., Kmetec, S., Duh, D., & Turk, S. S. (2021). Reduced susceptibility and increased resistance of bacteria against disinfectants: A systematic review. In *Microorganisms* (Vol. 9, Issue 12). https://doi.org/10.3390/microorganisms9122550
Sansone, C., Van Houte, J., Joshipura, K., Kent, R., & Margolis, H. C. (1993). The Association of Mutans Streptococci and Non-Mutans Streptococci Capable of Acidogenesis at a Low pH with Dental Caries on Enamel and Root Surfaces. Journal of Dental Research, 72(2). https://doi.org/10.1177/00220345930720020701
Shin, J. M., Ateia, I., Paulus, J. R., Liu, H., Fenno, J. C., Rickard, A. H., & Kapila, Y. L. (2015). Antimicrobial nisin acts against saliva derived multi-species biofilms without cytotoxicity to human oral cells. Frontiers in Microbiology, 6(JUN). https://doi.org/10.3389/fmicb.2015.00617
Simón-Soro, A., Guillen-Navarro, M., & Mira, A. (2014). Metatranscriptomics reveals overall active bacterial composition in caries lesions. In Journal of Oral Microbiology (Vol. 6, Issue 1). https://doi.org/10.3402/jom.v6.25443
Warreth, A. (2023). Dental Caries and Its Management. In International Journal of Dentistry (Vol. 2023). https://doi.org/10.1155/2023/9365845
Yuan, X., Long, Y., Ji, Z., Gao, J., Fu, T., Yan, M., Zhang, L., Su, H., Zhang, W., Wen, X., Pu, Z., Chen, H., Wang, Y., Gu, X., Yan, B., Kaliannan, K., & Shao, Z. (2018). Green Tea Liquid Consumption Alters the Human Intestinal and Oral Microbiome. Molecular Nutrition and Food Research, 62(12). https://doi.org/10.1002/mnfr.201800178
WHO: "Global Oral Health Status Report: Towards Universal Health Coverage for Oral Health by 2030. Summary of the WHO European Region"
I. Kleinberg. (1967). Effect of urea concentration on human plaque pH levels in situ. , 12(12), 0-1484. doi: 10.1016/0003-9969(67)90183-5

## Claims

1. A dental care and/or an oral care product,
**characterized in that** said product
i) comprises between 0.0039% and 0.0078% by weight of nisin; and
ii) establishes and/or maintains a pH of at least 4.5 *in situ,* wherein said *in situ* is preferably at the oral and/or dental application site in an oral cavity, further preferably at a tooth surface.

2. The product of claim 1, wherein said product does not comprise more than 15% by weight of sodium bicarbonate, wherein said product preferably does not comprise sodium bicarbonate.

3. The product of claim 1 or claim 2, wherein said nisin concentration by weight does not provide for at least one of the following:
i) an anti-bacterial effect, including an anti-Mutans streptococci effect, wherein said Mutans streptococci are selected from *Streptococcus mutans* and *Streptococcus sobrinus,*
ii) a dysbacteriosis effect,
iii) a tooth or tooth surface enamel demineralization effect.

4. The product of claim 3, wherein said anti-bacterial effect is measured by the *in-vitro* test applied in Example 1 of the specification.

5. The product of any of the preceding claims, wherein said product is further defined by at least one of the following:
i) said product controls biofilms, preferably Mutans streptococci comprising biofilms, on said tooth surface,
ii) said product inhibits the growth of biofilms, preferably Mutans streptococci comprising biofilms, on said tooth surface,
iii) said product removes biofilms, preferably Mutans streptococci comprising biofilms, from said tooth surface.

6. The product of claim 5, wherein said control of biofilms, inhibiting the growth of biofilms and removing of biofilms is more effectively than a control product identical in composition except for the nisin compound missing in said control product.

7. The product of any of the preceding claims, wherein said product establishes and/or maintains at least for and/or within 30 minutes upon *in situ* application of said product a pH above 4.5, preferably above 5.0, further preferably above 5.5 or above 6.0.

8. The product of claim 7, wherein said pH is measured at the at the oral or dental application site in the oral cavity, further preferably at the tooth surface.

9. The product of any of the preceding claims, wherein
i) said product aids in cleaning teeth and/or the oral cavity, wherein said product is preferably selected from the group consisting of a mouthwash, a toothpaste, an oral foam or an oral gel,
and/or
ii) said product is a formulation selected from the following: film, aerosol, suspension, solution, tincture, cream, paste, lotion, ointment, gel, powder, granulate.

10. Use of the product of any of the preceding claims for non-medical and/or aesthetic use.

11. The use of claim 10, wherein said use comprises preventing destruction of a subject's teeth enamel.

12. The use of claim 11, wherein said teeth enamel destruction results in non-aesthetic teeth features, including cracks and chips, discoloration and/or yellow staining, indentations, rough edges, shiny spots, translucent or fractured teeth.

13. The product of any of claims 1-9 for use as a medicament.

14. The product of any of claims 1-9 for use in the prophylaxis or treatment of an oral disease in a subject, wherein said oral disease is selected from the group consisting of caries, periodontitis, fluorosis, hyperesthesia, gingivitis; stomatitis, halitosis, sensitive calculus.

15. The product for use of claim 14, wherein said oral disease is dental caries.
